(19) **Europäisches Patentamt / European Patent Office / Office européen des brevets**

(11) **EP 3 608 397 A1**

(12) # EUROPEAN PATENT APPLICATION
published in accordance with Art. 153(4) EPC

(43) Date of publication:
**12.02.2020 Bulletin 2020/07**

(21) Application number: **18781102.1**

(22) Date of filing: **30.03.2018**

(51) Int Cl.:
*C12N 1/20* (2006.01)     *A01C 1/00* (2006.01)
*A01G 7/00* (2006.01)     *A01N 63/00* (2020.01)
*A01P 3/00* (2006.01)     *A01P 21/00* (2006.01)

(86) International application number:
**PCT/JP2018/013857**

(87) International publication number:
**WO 2018/186307 (11.10.2018 Gazette 2018/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.04.2017 JP 2017074841**

(71) Applicants:
• **Gifu University**
  **Gifu-shi, Gifu 501-1193 (JP)**
• **Kumiai Chemical Industry Co., Ltd.**
  **Taito-ku**
  **Tokyo 110-8782 (JP)**

(72) Inventors:
• **SHIMIZU, Masafumi**
  **Gifu-shi**
  **Gifu 501-1193 (JP)**
• **MAREK Caled Malianne**
  **Gifu-shi**
  **Gifu 501-1193 (JP)**
• **SUZUKI, Satomi**
  **Tokyo 110-8782 (JP)**
• **OZAKI, Koichi**
  **Tokyo 110-8782 (JP)**

(74) Representative: **Fédit-Loriot**
  **38, avenue Hoche**
  **75008 Paris (FR)**

(54) **STRAIN BELONGING TO GENUS MITSUARIA AND MICROBIAL PESTICIDE USING SAID STRAIN**

(57)     Provided herein is a non plant-pathogenic strain that can stably exhibit a bacterial plant disease control effect at the actual site of crop production, and that can be safely used as a biopesticide against plant. A bacterial plant disease control agent using such a strain is also provided, among others. In the present invention, viable bacteria of a strain belonging to genus Mitsuaria, which was not known to have a control effect against bacterial plant disease, or a culture containing the viable bacteria of such a strain are used as an active component, and the invention provides bacterial plant disease control agents such as a vegetable wilt disease control agent, and a canker control agent.

**Description**

Technical Field

The present invention relates to bacterial strains of genus Mitsuaria, and to bacterial plant disease control agents and plant growth regulators using such strains, among others.

Background Art

Control of crop diseases (plant diseases) caused by pathogenic bacteria is a very important issue among farmers around the globe. Cultural control and use of antimicrobial agents are two traditional approaches to the management of plant disease. However, some bacterial plant diseases are difficult to control by traditional methods, cultural control and antimicrobial agents included.

An example of such plant diseases is bacterial wilt, which is a soil-borne disease caused by Ralstonia solanacearum, a soil-borne bacterium. In plants such as tomato, eggplant, green pepper, and potato, a plant infected with this bacterial wilt-causing bacterium dies after rapid wilting that occurs throughout the plant, and crop production is greatly affected by this bacterium. Ralstonia solanacearum is a widely distributed pathogen, mainly in tropical, subtropical, and temperate regions of the world, infecting over 200 plant species, particularly the Solanaceae plants, and damaging the crops. Indeed, bacterial wilt is one of the serious, important issues in farming.

To date, a large number of control techniques against bacterial wilt disease have been proposed. Examples of such techniques currently in use include use of chemical pesticides, soil disinfection such as by solar disinfection or reductive disinfection, and cultivation or breeding of resistant rootstock varieties. The bacterial wilt-causing bacteria are able to survive for extended time periods deep in the soil (about 50 cm to 1 m from surface) even when the soil surface is successfully disinfected by means of, for example, soil disinfection. Currently, no soil disinfectant is available that can reach into the soil this deep, and complete removal of bacteria from soil remains a difficult task indeed. The resistance of resistant varieties is not perfect either, and fails to exhibit sufficient effect depending on the environmental conditions. There has been proposed another type of pesticide, for example, such as the bacterial wilt resistance inducer disclosed in PTL 1. However, the efficacy of such pesticides is also not sufficient.

As a countermeasure, a control method is proposed that makes use of a biopesticide to inhibit the development of a plant disease through antagonistic action against pathogens present in the soil. Control of bacterial wilt-causing bacteria with microbial pesticides has been studied with regard to strains of genera Pseudomonas, Ralstonia, Acinetobacter, and Pythium (for example, see NPL 1 for microbial pesticides using the Acinetobacter GEBT349 strain). However, control by these biopesticides is not sufficiently effective either. There have been attempts to control bacterial wilt disease by feeding compost containing different microorganisms. However, the effectiveness of this method is unclear, and many of the previous attempts have failed. The foregoing methods also require that the antagonistic microorganisms must colonize in the soil, the rhizosphere and the plant. However, it is often difficult to consistently produce a stable effect in widely changing conditions of soil. Another difficulty is that colonization and proliferation are difficult to achieve with strains that are not pathogenic to plant. Indeed, this is one of the reasons for the previous failure to establish a control method using biopesticides.

Meanwhile, canker is another bacterial plant disease that kills tomato, Japanese apricot, kiwifruit, and citrus fruits by infecting these plants. Canker is a soil borne disease caused by Clavibacter michiganensis and other bacteria. The pathogens are said to survive for longer than 3 years in soil, making the disease very difficult to control as with the case of bacterial wilt.

Given these technical backgrounds, the industry requires development and commercialization of a bacterial disease control agent that makes use of a microorganism capable of colonizing and proliferating in the soil or plant without pathogenicity to plant but showing sufficient levels of basic activity, and that can be safely used as an pesticide with the effect to sufficiently control (both practically and stably) bacterial wilt, canker, and other bacterial plant diseases that are difficult to control by traditional approach.

Prior Art

Citation List

Patent Literature

PTL 1: JP-A-2012-211124

Non Patent Literature

NPL 1: Japanese Journal of Phytopathology, Vol. 82 (2016), No. 3, p. 246

Summary of Invention

Technical Problem to be solved by Invention

It is an object of the present invention to provide a non plant-pathogenic strain that can stably exhibit a bacterial plant disease control effect at the actual site of crop production, and that can be safely used as a biopesticide against plant, and to provide a bacterial plant disease control agent using such a strain, among others.

Means for Solving

The prevent inventors conducted intensive studies to achieve the foregoing objects, and found that a strain belonging to genus Mitsuaria, which was not known to have a control effect against bacterial plant disease, can produce a practical effect for the control of bacterial plant disease, and that the strain is safe to use at the actual site of crop production. The present invention has been completed on the basis of these findings.
Specifically, embodiments of the present invention are as follows.

(1) A strain belonging to genus Mitsuaria and having a bacterial plant disease control effect.
(2) The strain according to item (1), wherein the strain belongs to Mitsuaria chitosanitabida.
(3) A TWR114 strain of Mitsuaria chitosanitabida (NITE BP-02445).
(4) A bacterial plant disease control agent which comprises viable bacteria of the strain of any one of items (1) to (3) or a culture containing said viable bacteria as an active component.
(5) The agent according to item (4), wherein the agent is a vegetable wilt disease control agent and/or a canker control agent (for example, a vegetable bacterial canker control agent).
(6) The agent according to item (4) or (5), wherein the agent is a control agent against disease of Solanaceae plant.
(7) A bacterial plant disease control method comprising the step of contacting viable bacteria of the strain of any one of items (1) to (3) or contacting a culture containing said viable bacteria to a plant and/or a soil (particularly, the rhizosphere) .
(8) The method according to item (7), wherein the method controls a vegetable wilt and/or a canker (for example, a vegetable bacterial canker).
(9) The method according to item (7) or (8), wherein the method controls a Solanaceae plant disease.
(10) A plant growth regulator that comprises viable bacteria of the strain of any one of items (1) to (3) or a culture containing said viable bacteria as an active component.
(11) The regulator according to item (10), wherein the regulator is a vegetable seed germination promoter and/or a vegetable growth promoter.
(12) A method for regulating plant growth, comprising the step of contacting viable bacteria of the strain of any one of items (1) to (3) or contacting a culture containing said viable bacteria to a plant and/or a soil (particularly, the rhizosphere).
(13) The method according to item (12), wherein the method promotes germination of a vegetable seed and/or vegetable growth.

Advantageous Effects of Invention

According to the present invention, a bacterial plant disease control agent can be provided that can be safely used at the actual site of crop production with the notable effect (both practical and stable) to control bacterial wilt, bacterial canker, and other bacterial plant diseases that are difficult to control by traditional approach, among others. The present invention can also provide a plant growth regulator, among others.

Brief Description of Drawings

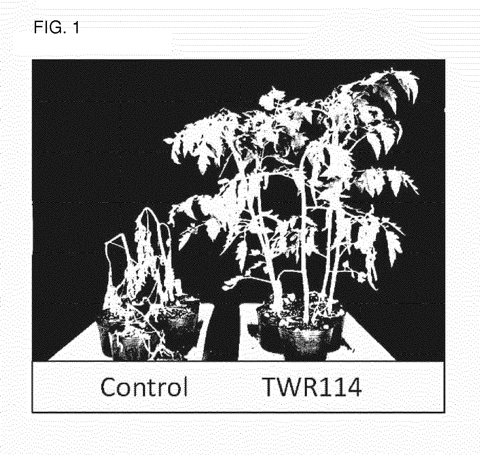

FIG. 1 shows pictures (photograph substituted for drawing) of tomato stocks after 14 days from inoculation of a causative bacterium of tomato bacterial wilt in a test conducted to confirm the effectiveness to control bacterial wilt of tomato in Example 1, in which the five stocks on left-hand side represent an untreated group (control), and the five stocks on the right-hand side represent a TWR114 strain-treated group (TWR114).

Description of Embodiments

In the present invention, a strain belonging to genus Mitsuaria, for example, a strain of Mitsuaria chitosanitabida is used as an active component of a bacterial plant disease control agent, among others. As used herein, "bacterial plant disease" means damage to useful plants in a plant disease caused by bacterial pathogens, and excludes damage caused by a plant disease involving other pathogens such as filamentous fungi and viruses. Previously, Mitsuaria bacteria were not known to have any bacterial plant disease control effect, and there is no known species of Mitsuaria that is pathogenic to plant.

Particularly preferred for use as a Mitsuaria strain in the present invention is a strain isolated from the rhizosphere soil of the green onion in the campus of Gifu University (1-1, Yanagido, Gifu City, Gifu), and that was identified as a novel non plant-pathogenic strain TWR114 belonging to Mitsuaria chitosanitabida, or a mutant strain of characteristics equivalent to the characteristics of the TWR114 strain, after bacteriological studies and a phylogenetic analysis using the genome sequence of 16S rRNA gene. A test conducted with an API 20NE kit (Sysmex bioMérieux Co., Ltd.) confirmed that the TWR114 strain had the following bacteriological characteristics.

(A) Morphological Characteristics

Morphology: Bacillus
Size: 0.9 to 1.9 $\mu$m in width, 0.9 to 3.0 $\mu$m in length
Mobility: +

(B) Culture Characteristics

Color of colony: Beige to pale pink
Bouillon agar plate culture: Beige to pale pink colonies with glossy surface

(C) Physiological Characteristics

Gram staining: -
Nitrate reduction: +
Indole production (triptophan): -
Glucose fermentation: -
Arginine dihydrolase: -
Urease: -
Hydrolysis ($\beta$-glucosidase): -
Hydrolysis (protease): +
Optimum growth pH: neutral range
Optimum growth temperature: 30 to 35°C
Assimilation (glucose): +
Assimilation (arabinose): +
Assimilation (mannose): +
Assimilation (mannitol): -
Assimilation (N-acetyl-glucosamine): +
Assimilation (maltose): +
Assimilation (potassium gluconate): +
Assimilation (capric acid): -
Assimilation (adipic acid): -
Assimilation (maleate): +
Assimilation (trisodium citrate): -
Assimilation (phenyl acetate): -

The TWR114 strain was first deposited in The National Institute of Technology and Evaluation, NITE Patent

EP 3 608 397 A1

Organism Depositary (2-5-8, Kazusa-Kamatari, Kisarazu-shi, Chiba, 292-0818, Japan) on March 17, 2017. Then, a request for transfer to an international depositary authority was received on January 22, 2018. The international deposition number is NITE BP-02445.

Any medium can be used for culture of a strain belonging to genus Mitsuaria, provided that it allows the strain to grow. Examples include common media such as bouillon medium, and media containing glucose, peptone, and yeast extract. The medium may be a liquid medium, or a solid medium such as an agar-supplemented slant or plate medium.

As a carbon source of the medium, all of sources can be utilized which the strains belonging to genus Mitsuaria are able to assimilate. Specific examples include various synthetic and natural carbon sources that can be utilized by strains of genus Mitsuaria, such as glucose, arabinose, mannose, starch hydrolysate, and molasses. The nitrogen source in medium may be chosen from a variety of synthetic and natural products that can be utilized by the strains, including, for example, organic nitrogen-containing products such as peptone, meat extract, yeast extract, and soybean meal. Additionally, trace amounts of nutrient sources may be added according to the customary method of microbial culture, as required. Examples of such nutrient sources include inorganic salts such as common salt and phosphates; metal salts such as calcium, magnesium, and iron salts; vitamins, amino acids, and nucleic acid-related substances. It is also possible to add an additive, for example, such as a defoaming agent, as required.

The strains belonging to the genus Mitsuaria may be cultured under aerobic conditions, for example, such as shaking culture or aeration culture. The culture conditions are not limited to these, and the strains are cultured for 1 to 4 days, preferably 2 to 3 days at a temperature of 20 to 40°C, preferably 30 to 35°C, at a pH of 5 to 8, preferably 6 to 7.

After cultivation in the manner described above, the strain belonging to the genus Mitsuaria can be used as an active component of, for example, a bacterial plant disease control agent in the form of a culture containing viable bacteria, without being separated from the culture. Alternatively, the viable bacteria may be separated from the culture using an ordinary method, for example, such as by membrane separation or centrifugation, and the bacteria separated from the culture, after optional washing, may be used as an active component either as it is or after treatment (for example, the bacteria separated from culture may be used as a mixture with other components) . It is also possible to use the bacterial culture or separated viable bacteria in the form of a dried product obtained by using a technique such as freeze drying or spray drying, or in the form of a diluted product obtained by diluting the cultured bacteria or separated bacteria of liquid or solid form. The cultured bacteria or separated bacteria also may be used in the form of various preparations produced by mixing various additives using traditional methods of producing pesticide formulations. Examples of such preparations include granular formulations, emulsions, wettable powders, and flowable formulations.

The concentration of viable bacteria contained in a bacterial plant disease control agent of the present invention is not particularly limited, as long as the bacterial plant disease control agent can produce the desired effect. However, because a bacterial concentration that is too low often fails to produce sufficient results, and a bacterial concentration that is too high is wasteful of bacteria, the bacterial concentration is appropriately adjusted in a range of $1 \times 10^5$ to $1 \times 10^{10}$ cfu/ml, desirably $1 \times 10^6$ to $1 \times 10^9$ cfu/ml, in the case of, for example, a liquid preparation. As used herein, "cfu" means colony forming unit. When using a culture, the culture may be appropriately designed according to these ranges of viable bacteria concentration.

The present invention exhibits desirable control effect against a wide range of bacterial plant diseases, including, for example, bacterial wilt of Solanaceae plants (e.g., eggplant, tomato, green pepper, paprika, and potato) and Cucurbitaceae plants (e.g., cucumber, and bitter gourd) caused by plant pathogenic Ralstonia solanacearum bacteria, and canker of tomato, Japanese apricot, kiwifruit, and citrus fruits (e.g., oranges, grapefruits, mandarin, and aromatic citrus fruits) caused by bacterial pathogens such as Clavibacter michiganensis, Pseudomonas syringae, and Xanthomonas citri. A particularly notable characteristic of the present invention is that the invention is highly effective against bacterial wilt of Solanaceae plant (Solanaceae crops), and bacterial canker of tomato.

As used herein, "control" means circumventing these and other plant diseases by preventing, for example, infections of crops (plants) by bacteria that cause disease in these crops and plants.

The present invention, in addition, exhibits a plant growth regulating effect promoting, for example, germination of seeds in Solanaceae and Brassicaceae crops (e.g., napa, cabbage, white radish, white turnip, broccoli, cauliflower, *bok choy, komatsuna,* and *mizuna*), or promoting growth of roots, stems, and leaves of these plants. That is, the present invention also has use as a plant growth regulator.

A plant disease control agent according to the present invention may be applied as it is, or after being diluted with, for example, water. Use of the plant disease control agent as a pesticidal formulation is not particularly limited, and, for example, the plant disease control agent may be directly applied to crops and seeds by being sprayed against crops and seeds or by soaking crops or seeds in the plant disease control agent. As another example, the plant disease control agent may be sprayed onto soil, or may be added to water or a fertilizer applied to crops and soil. As another example, the plant disease control agent may be applied to farm equipment. Preferably, the pesticidal formulation is added to water applied to soil, or applied to farm equipment. That is, a bacterial plant disease control agent according to the present invention inhibits various bacterial plant diseases or regulates plant growth by being present on a plant, for example, on the root, stem, leaves, or seeds of a plant, or on a soil used for cultivation.

A pesticidal formulation of the present invention is used in amounts that depend on factors such as the type of the crop of interest, the type of the disease to be controlled, the method used to apply the formulation, patterns of disease occurrence, extent of damage, environmental conditions, and the form of formulation. Accordingly, there is no specific amount, and the pesticidal formulation is preferably used in appropriately adjusted amounts. As an example, in the case of a liquid formulation, the pesticidal formulation is used in an amount of 1 ml to 10 L, preferably 2 ml to 3 L, further preferably 2.5 ml to 1 L per stock of a crop plant. The time to use the pesticidal formulation also depends on factors such as the type of the disease to be controlled, and the form of pesticidal formulation. Preferably, the pesticidal formulation is applied before and/or after sowing when its effect is desired in seeds. When the effect is desired in growing plants, the pesticidal formulation is appropriately applied before planting and/or within about 4 weeks after planting. In the present invention, the emergence of resistant bacteria is not of concern, and the microbial pesticide of the present invention can be continuously used for several days, or can be used also for monocropping.

A bacterial plant disease control agent according to the present invention may be used with one or more chemical pesticides selected from the following compounds, as required:

inorganic copper compounds, for example, such as basic copper sulfate, anhydrous copper sulfate, copper (II) hydroxide, and basic copper chloride;

organic copper compounds, for example, such as organic copper, and cupric nonylphenol sulfonate;

inorganic sulfur compounds, for example, such as sulfur, and total sulfide sulfur;

organic sulfur compounds, for example, such as zineb, maneb, propineb, thiadiazin, thiuram, and polycarbamate;

anilinopyrimidine compounds, for example, such as cyprodinil, pyrimethanil, and mepanipyrim;

phenylpyrrole compounds, for example, such as fludioxonil;

organic chlorine compounds, for example, such as chlorothalonil, captan, triazin, fluazinam, sulfenic acid, and fthalide;

hydrogen carbonates, for example, such as sodium bicarbonate, and potassium bicarbonate;

organic phosphorus compounds, for example, such as EDDP, fosetyl, tolclofos-methyl, and IBP;

benzimidazole compounds, for example, such as carbendazim, thiophanate-methyl, thiabendazole, benomyl, and fuberidazole;

dicarboxyimide compounds, for example, such as iprodione, procymidone, and vinclozolin;

azole compounds, for example, such as fenbuconazole, simeconazole, diclobutrazol, triticonazole, ipuconazole, fluconazole, myclobutanil, penconazole, bitertanol, bromuconazole, oxpoconazole, cyproconazole, difenoconazole, diniconazole, epoxiconazole, fenbuconazole, fluquinconazole, flusilazole, flutriafol, hexaconazole, imibenconazole, metconazole, propiconazole, cyproconazole, tebuconazole, tetraconazole, triadimefon, and triadimenol;

imidazole compounds, for example, such as triflumizole, prochloraz, imazalil, and pefurazoate;

piperazine compounds, for example, such as triforine;

morpholine compounds, for example, such as fenpropimorph, tridemorph, and fenpropidine;

hydroxypyrimidine compounds, for example, such as ethirimol, and dimethirimol;

guanidine compounds, for example, such as iminoctadine acetate, iminoctadine albesilate, and guazatine;

acid amide compounds, for example, such as oxycarboxin;

benzoanilide compounds, for example, such as mepronil, diclomezine, flutolanil, pencycuron, furametpyr, and thifluzamide;

acylalanine compounds, for example, such as oxadixyl, and metalaxyl;

methoxyacrylate compounds, for example, such as azoxystrobin, kresoxim-methyl, metominostrobin, trifloxystrobin, picoxystrobin, pyraclostrobin, and orysastrobin;

quinoxaline compounds, for example, such as quinomethionate;

hydroxyanilide compounds, for example, such as fenhexamid;

cyanoacetamide compounds, for example, such as cymoxanil;

cyanoimidazole compounds, for example, such as cyazofamid; and

famoxadone, spiroxamine, triazoxide, pyrazophos, fluoroimide, dimethoroph, iprovalicarb, fenamidone, ethaboxam, cyflufenamid, dithianon, carpropamid, probenazole, methasulfocarb, pyroquilon, hydroxyisoxazole, tricyclazole, diflumetorim, phenazine oxide, isoprothiolane, oxolinic acid, acibenzolar-S-methyl, quinoxyfen, benthiavalicarb-isopropyl, and tiadinil. In this case, the mixture may be applied after being prepared under the conditions that have only little effect on the active component strain. The bacterial plant disease control agent and one or more chemical pesticides may be applied at the same or different times.

As described above, viable bacteria of strains of genus Mitsuaria, for example, the TWR114 strain of Mitsuaria chitosanitabida, or a culture of such viable bacteria are used as an active component to provide a pesticidal formulation that, in addition to a plant growth regulating effect, produces a notable effect for the control of vegetable wilt, canker, and other bacterial plant diseases, and that can be safely used at the actual site of crop production.

The following describes the present invention through Examples. It is to be noted that the present invention is not limited to the Examples below, and various modifications are possible within the technical idea of the present invention.

Example 1

(Test 1 for Confirmation of Control Effect on Bacterial Wilt of Tomato)

A tomato (variety: Ponderosa) was grown to the fourth compound-leaf stage in a plastic pot (diameter: 9 cm) filled with a planting soil (150 g of planting soil in the bottom layer, 20 g of river sand in the middle layer, and 150 g of planting soil in the top layer). Separately, 30 ml of a suspension of Mitsuaria chitosanitabida TWR114 strain was mixed with 70 ml of sterile water. The plant in the plastic pot was then treated with the suspension by applying it with water from the bottom of the pot. After being kept in a 30°C greenhouse for 3 days, the tomato plant was inoculated with 100 ml of a suspension of causative bacteria of tomato bacterial wilt by supplying water from the bottom with the bacterial suspension in the same manner. For comparison, some tomato stocks were treated only with a suspension of causative bacteria of tomato bacterial wilt supplied with water from the bottom using the same procedure (untreated group). The suspension of TWR114 strain was prepared by growing the bacteria by shaking culture at 27°C at 120 rpm for 48 hours using an NB medium (meat extract 0.5%, peptone 1.5%, sodium chloride 0.5%, potassium monohydrogen phosphate 0.5%, pH 7.0), and collecting cells by centrifugation (5,000 $\times$ g, 15 min), followed by suspension in sterile water. This was repeated two times to obtain the suspension of TWR114 strain. The suspension prepared had an absorbance at 600 nm (OD600) of 1.0 ($3 \times 10^9$ cfu/ml). The suspension of causative bacteria of tomato bacterial wilt was prepared by diluting the bacteria with $MgCl_2$ (10 mM) after growing the cells in a 24-hour shaking culture using CPG medium, and collecting the bacteria by centrifugation. The suspension prepared had an absorbance at 600 nm (OD600) of 1.0 (about $1 \times 10^9$ cfu/ml).

A total of ten treated stocks in each group were investigated by counting the number of pots that showed bacterial wilt of tomato after 7 days from the inoculation of the causative bacteria of tomato bacterial wilt, and the percentage of diseased stocks was calculated. Five of the stocks from each group were visually inspected and photographed after 14 days from the inoculation of the causative bacteria of tomato bacterial wilt.

The test results are shown in Table 1 (percentage of diseased stocks) and in FIG. 1 (pictures) . In contrast to the untreated group in which all of the stocks developed bacterial wilt of tomato, the TWR114 strain-treated group had much lower percentages of diseased stocks, and the visual inspection confirmed that the bacterial wilt of tomato was clearly inhibited, demonstrating that the strain has a strong control effect against bacterial wilt of tomato.

[Table 1]

|  | Treatment concentration (cfu/ml) | Treatment amount (ml/pot) | Healthy | Diseased | Percentage of diseased stocks |
|---|---|---|---|---|---|
| TWR114 | $3 \times 10^9$ | 30 | 6 | 4 | 40 |
| Untreated group |  |  | 0 | 10 | 100 |

Example 2

(Test 2 for Confirmation of Control Effect on Bacterial Wilt of Tomato)

A tomato (variety: Ponderosa) was grown to the fourth compound-leaf stage in a pot (9 cm $\times$ 9 cm) filled with a gardening soil. A suspension of TWR114 strain (30 ml) was then poured onto the soil. After being kept in a 30°C greenhouse for 4 days, the plant was inoculated with 50 ml of a suspension of causative bacteria of tomato bacterial wilt by pouring the suspension using the same procedure. For comparison, some stocks were treated only with a suspension of causative bacteria of tomato bacterial wilt poured onto the soil (untreated group), while a 10-ml suspension of the Acinetobacter GEBT349 strain described in NPL 1 was sprayed onto the stems and leaves of other stocks (control group). For the preparation of the TWR114 strain suspension and GEBT349 strain suspension, each suspension was prepared by growing the bacteria by shaking culture at 27°C at 120 rpm for 48 hours using an NB medium (meat extract 0.5%, peptone 1.5%, sodium chloride 0.5%, monopotassium phosphate 0.5%, pH 7.0), and collecting cells by centrifugation (5, 000 $\times$ g, 15 min), followed by suspension in sterile water. This was repeated two times to obtain the suspensions. The suspensions prepared had an absorbance at 600 nm (OD600) of 1.0 ($3 \times 10^9$ cfu/ml). The suspension of causative bacteria of tomato bacterial wilt was prepared by diluting the grown and centrifuged cells 100 times with distilled water after 24-hour shaking culture using YP medium.

The three stocks after each treatment were respectively examined with regard to the extent of bacterial wilt of tomato after 10 days from the inoculation of the causative bacteria of tomato bacterial wilt, using the following index criteria. The results were used to calculate disease severity and control effiacy.

<Disease Index>

0: No disease
1: Wilt occurred in some leaflets
2: Wilt occurred in less than half of compound leaves
3: Wilt occurred in at least half of compound leaves
4: Withered and died

<Disease severity and Control Efficacy>

$$\text{Disease severity} = \Sigma \text{ (disease index} \times \text{ number of stocks}$$

$$\text{with corresponding index)/(number of stocks investigated} \times 4)$$

$$\times \; 100$$

$$\text{Control efficacy} = 100 - \text{ (disease severity of treated}$$

$$\text{group/disease severity of untreated group)} \times 100$$

The test results are presented in Table 2 below. The untreated group had a disease severity of 58.3. The control group had a disease severity of 41.7, and a control efficacy of 28.6. In contrast, the TWR114 strain-treated group had a much lower disease severity (8.3) and a much higher control efficacy (85.7), demonstrating that the TWR114 strain has a notably strong control effect against bacterial wilt of tomato, even when compared to the control group.

[Table 2]

| Tested bacterial strain | Treatment method | Treatment concentration (cfu/ml) | Treatment amount (ml/pot) | Disease severity | Control efficacy |
|---|---|---|---|---|---|
| TWR114 | Pour on soil | $3 \times 10^9$ | 30 | 8.3 | 85.7 |
| GEBT | Spray on stems and leaves | $3 \times 10^9$ | 10 | 41.7 | 28.6 |
| Untreated | | | | 58.3 | |

Example 3

(Test to Confirm Control Effect on Bacterial Wilt of Potato)

A potato (variety: Dejima) was grown to the fourth compound-leaf stage in a plastic pot (diameter: 9 cm) filled with a gardening soil. A suspension of TWR114 strain (30 ml) was then poured into the pot. After being kept in a 30°C greenhouse for 4 days, the plant was inoculated with 50 ml of a suspension of causative bacteria of potato bacterial wilt by pouring the suspension using the same procedure. For comparison, some stocks were treated only the suspension of causative bacteria of potato bacterial wilt poured onto the soil (untreated group), while a 10-ml suspension of Acinetobacter GEBT349 was sprayed onto the stems and leaves of other stocks (control group). For the preparation of the TWR114 strain suspension and GEBT349 strain suspension, each suspension was prepared by growing the bacteria by shaking culture at 30°C at 120 rpm for 24 hours using an NB medium (meat extract 0.5%, peptone 1.5%, sodium chloride 0.5%, potassium monohydrogen phosphate 0.5%, pH 7.0), and collecting cells by centrifugation (5,000 × g, 10 min), followed by suspension in sterile water. This was repeated two times to obtain the suspensions. The suspensions prepared had an absorbance at 600 nm (OD600) of 1.0 ($3 \times 10^9$ cfu/ml). The suspension of causative bacteria of potato bacterial wilt was prepared by diluting the grown bacteria 100 times with distilled water after 24-hour shaking culture using YP medium.

The three stocks after each treatment were respectively examined with regard to the extent of bacterial wilt of potato after 14 days from the inoculation of the causative bacteria of potato bacterial wilt, using the following index criteria. The results were used to calculate disease severity and control efficacy.

<Disease Index>

0:    No disease
1:    Wilt occurred in some leaflets
2:    Wilt occurred in less than half of compound leaves
3:    Wilt occurred in at least half of compound leaves
4:    Withered and died

Disease Severity and Control Efficacy>

$$\text{Disease severity} = \Sigma \text{ (disease index} \times \text{number of stocks}$$

$$\text{with corresponding index)/(number of stocks investigated} \times 4)$$

$$\times 100$$

$$\text{Control efficacy} = 100 - \text{(disease severity of treated}$$

$$\text{group/disease severity of untreated group)} \times 100$$

The test results are presented in Table 3 below. The untreated group had a disease severity of 50, whereas the control group had a disease severity of 75.0, higher than that of the untreated group. In contrast, the TWR114 strain-treated group had a disease severity of 16.7, and a control efficacy of 66.7, demonstrating that the TWR114 strain also has a notably strong control effect against bacterial wilt of potato.

[Table 3]

| Tested bacterial strain | Treatment method | Treatment concentration (cfu/ml) | Treatment amount (ml/pot) | Disease severity | Control efficacy |
|---|---|---|---|---|---|
| TWR114 | Pour on soil | $3 \times 10^9$ | 30 | 16.7 | 66.7 |
| GEBT | Spray on stems and leaves | $3 \times 10^9$ | 10 | 75 | 0 |
| Untreated group | | | | 50 | |

Example 4

(Test to Confirm Control Effect on Bacterial Canker of Tomato)

A tomato (variety: Momotaro 8) was grown to the fourth compound-leaf stage in a plastic pot (diameter: 9 cm) filled with a planting soil, as in Example 1. Separately, a suspension of causative bacteria of tomato bacterial canker was sprayed once onto scissors with a mister, followed by spraying of a suspension of TWR114 strain to both sides of the scissors, once on each side. The first true leaf of tomato was cut at the base of the petiole using the scissors, and the leaf was grown in a 28°C glass green house for 1 month. For comparison, only a suspension of causative bacteria of tomato bacterial canker was sprayed once onto scissors. The first true leaf of tomato was cut at the base of the petiole using the scissors, and the leaf was grown in a 28°C glass green house for 1 month (untreated group). The TWR114 strain suspension was prepared using the same method used in Example 1. The suspension of causative bacteria of tomato bacterial canker was prepared by growing the bacteria by shaking culture with PSB medium for 4 days, and collecting cells by centrifugation, followed by suspension in sterile water. The suspension was used at a concentration of about $6 \times 10^8$ cfu/ml.

Each stock after treatment was respectively examined with regard to the extent of disease in compound leaves after 28 days from the inoculation of the causative bacteria of tomato bacterial canker, using the following index criteria. The results were used to calculate disease severity and control efficacy.

<Disease Index>

0: No symptoms of disease
1: Necrosis and symptoms of wilt were observed in part of compound leaves
2: Necrosis and wilt occurred in most part of compound leaves

<Disease Severity and Control Efficacy>

$$\text{Disease severity (\%)} = \{\Sigma \text{ (disease index} \times \text{number of compound leaves)/total number of compound leaves} \times 2\} \times 100$$

$$\text{Control efficacy} = \{1 - \text{(disease severity of treated group/disease severity of untreated group)}\} \times 100$$

The test results are presented in Table 4 below. In contrast to the untreated group that had a disease severity of 91.4, the TWR114 strain had a much lower disease severity (46.6) than the untreated group, and the control efficacy was high (49.0), demonstrating that the TWR114 strain also has excellent control effect against bacterial canker of tomato.

[Table 4]

| | Treatment concentration (cfu/ml) | Disease severity | Control efficacy |
|---|---|---|---|
| TWR114 | $1 \times 10^9$ | 46.6 | 49.0 |
| Untreated group | | 91.4 | |

Example 5

(Test to Confirm Germination Promoting Effect in Tomato Seed)

A filter paper was laid over two Petri dishes having a diameter of 60 mm, and sixty tomato seeds (variety: Ponderosa) were sowed on the filter paper of each Petri dish. After adding 2 ml of a TWR114 strain suspension or tap water, the plant was allowed to stand in an incubator maintained at 20°C during nighttime and 25°C during daytime. The TWR114 strain suspension was prepared by growing the bacteria by shaking culture at 27°C at 120 rpm for 6 days using an NB medium, and collecting cells by centrifugation ($5,000 \times$ g, 15 min), followed by suspension in sterile water. This was repeated two times to obtain the suspension. The suspension prepared had an absorbance at 600 nm (OD600) of 1.0.

The plants were visually inspected for a germination state of seeds after 6 days from sowing, and the germination rate was calculated.

The test results are presented in Table 5 below. In contrast to the tap water-added group (untreated group) that had a germination rate of 65.0%, the TWR114 strain-treated group had a greatly improved germination rate (85.0%) over the untreated group, demonstrating that the strain has a germination promoting effect in tomato seed.

[Table 5]

|  | No germination | Germination | Germination rate (%) |
|---|---|---|---|
| Mitsuaria chitosanitabida TWR114 | 9 | 51 | 85.0 |
| Tap water | 21 | 39 | 65.0 |

Example 6

(Test to Confirm Tomato Growth Promoting Effect)

A sterilized soil (planting soil:river sand:vermiculite = 1:1:1) was charged into a plastic pot having a diameter of 9 cm, and a tomato (variety: Ponderosa) was grown to the fourth compound-leaf stage in the soil. Separately, 30 ml of a suspension of TWR114 strain was mixed with 70 ml of sterile water. The plant in the plastic pot was then treated with the suspension by applying it with water from the bottom of the pot, and was kept in a 30°C greenhouse for 28 days with water being appropriately supplied from the bottom. For comparison, some stocks were treated only with water supplied from the bottom (untreated group) . The suspension of TWR114 strain was prepared using the same method used in Example 1.

After 27 days from the start of treatment, the plants were measured for the fresh weights of stem and leaf portions, and the fresh weight of root portions.

The test results are presented in Table 6 below. The results showed that the treatment with the TWR114 strain promotes growth at the root portion of tomato.

[Table 6]

|  | Treatment concentration (cfu/ml) | Treatment amount (ml/pot) | Fresh weight of stem and leaf (g) | Fresh weight of root portion (g) |
|---|---|---|---|---|
| TWR114 | $3 \times 10^9$ | 30 | 17.31 | 3.98 |
| Untreated group |  |  | 17.56 | 3.37 |

Example 7

(Test to Confirm Germination Promoting Effect in Cabbage Seed)

A filter paper was laid over two Petri dishes having a diameter of 60 mm, and, after adding 1 ml of tap water, thirty cabbage seeds (variety: Shikidori) were sowed on the filter paper of each Petri dish. After adding 1 ml of a TWR114 strain suspension or tap water, the plant was allowed to stand in an incubator maintained at 20°C during nighttime and 25°C during daytime. The TWR114 strain suspension was prepared by growing the bacteria by shaking culture at 27°C at 120 rpm for 6 days using an NB medium, and collecting cells by centrifugation (5,000 $\times$ g, 15 min), followed by suspension in sterile water. This was repeated two times to obtain the suspension. The suspension prepared had an absorbance at 600 nm (OD600) of 0.32.

The plants were visually inspected for a germination state of seeds after 6 days from sowing, and the germination rate was calculated.

The test results are presented in Table 7 below. In contrast to the tap water-added group that had a germination rate of 63.3%, the TWR114 strain-treated group had an improved germination rate (73.3%) over the untreated group, demonstrating that the strain has a germination promoting effect also in cabbage seed.

[Table 7]

|  | No germination | Germination | Germination rate (%) |
|---|---|---|---|
| Mitsuaria chitosanitabida TWR114 | 8 | 22 | 73.3 |

(continued)

|  | No germination | Germination | Germination rate (%) |
|---|---|---|---|
| Tap water | 11 | 19 | 63.3 |

As demonstrated above, a treatment with viable bacteria of the TWR114 strain was shown to enable control of bacterial plant diseases such as bacterial wilt of tomato, bacterial wilt of potato, and bacterial canker of tomato in practical applications. The TWR114 strain was also shown to promote plant growth, including promotion of seed germination in tomato and cabbage, and promotion of tomato growth.

The present invention can be summarized as follows.

The present invention is intended to provide a non plant-pathogenic strain that can stably exhibit a bacterial plant disease control effect at the actual site of crop production, and that can be safely used as a biopesticide against plant, and to provide a bacterial plant disease control agent using such the strain, among others.

In the present invention, viable bacteria of a strain belonging to genus Mitsuaria, which was not known to have a control effect against bacterial plant disease, or a culture containing the viable bacteria of such a strain are used as an active component, and the invention provides bacterial plant disease control agents such as a vegetable wilt disease control agent, and a canker control agent.

Reference to Deposited Biological Material

The deposition number of a microorganism that has been deposited in relation to the present invention is as follows.

(1) Mitsuaria chitosanitabida TWR114 strain (NITE BP-02445).

**Claims**

1.   A strain belonging to genus Mitsuaria and having a bacterial plant disease control effect.

2.   The strain according to claim 1, wherein the strain belongs to Mitsuaria chitosanitabida.

3.   A TWR114 strain of Mitsuaria chitosanitabida (NITE BP-02445).

4.   A bacterial plant disease control agent which comprises viable bacteria of the strain of any one of claims 1 to 3 or a culture containing said viable bacteria as an active component.

5.   The agent according to claim 4, wherein the agent is a vegetable wilt disease control agent and/or a canker control agent.

6.   The agent according to claim 4 or 5, wherein the agent is a Solanaceae plant control agent.

7.   A bacterial plant disease control method comprising the step of contacting viable bacteria of the strain of any one of claims 1 to 3 to a plant and/or a soil, or contacting a culture containing the viable bacteria to a plant and/or a soil.

8.   The method according to claim 7, wherein the method controls a vegetable wilt and/or a canker.

9.   The method according to claim 7 or 8, wherein the method controls a Solanaceae plant disease.

10.   A plant growth regulator that comprises viable bacteria of the strain of any one of claims 1 to 3 as an active component, or in which a culture containing the viable bacteria is contained as an active component.

11.   The regulator according to claim 10, wherein the regulator is a vegetable seed germination promoter and/or a vegetable growth promoter.

12.   A method for regulating plant growth, comprising the step of contacting viable bacteria of the strain of any one of claims 1 to 3 to a plant and/or a soil, or contacting a culture containing the viable bacteria to a plant and/or a soil.

13. The method according to claim 12, wherein the method promotes germination of a vegetable seed and/or vegetable growth.

FIG. 1

Control     TWR114

## INTERNATIONAL SEARCH REPORT

| International application No. |
|---|
| PCT/JP2018/013857 |

| A. CLASSIFICATION OF SUBJECT MATTER |
|---|
| Int.Cl. C12N1/20(2006.01)i, A01C1/00(2006.01)i, A01G7/00(2006.01)i, A01N63/00(2006.01)i, A01P3/00(2006.01)i, A01P21/00(2006.01)i |

According to International Patent Classification (IPC) or to both national classification and IPC

| B. FIELDS SEARCHED |
|---|
| Minimum documentation searched (classification system followed by classification symbols) |
| Int.Cl. C12N1/20, A01C1/00, A01G7/00, A01N63/00, A01P3/00, A01P21/00 |

| Documentation searched other than minimum documentation to the extent that such documents are included in the fields searched |
|---|
| Published examined utility model applications of Japan 1922–1996 |
| Published unexamined utility model applications of Japan 1971–2018 |
| Registered utility model specifications of Japan 1996–2018 |
| Published registered utility model applications of Japan 1994–2018 |

| Electronic data base consulted during the international search (name of data base and, where practicable, search terms used) |
|---|
| JSTPlus/JMEDPlus/JST7580(JDreamIII), CAplus/MEDLINE/EMBASE/BIOSIS/WPIDS(STN), PubMed |

### C. DOCUMENTS CONSIDERED TO BE RELEVANT

| Category* | Citation of document, with indication, where appropriate, of the relevant passages | Relevant to claim No. |
|---|---|---|
| X<br>A | SOMEYA, N. et al., Diversity of culturable chitinolytic bacteria from rhizospheres of agronomic plants in japan, Microbes Environ., 2011, vol. 26, no. 1, pp. 7-14, abstract, table 1 | 1-2, 4-13<br>3 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 18.06.2018 | 26.06.2018 |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

# EP 3 608 397 A1

## INTERNATIONAL SEARCH REPORT

International application No.

PCT/JP2018/013857

C (Continuation).   DOCUMENTS CONSIDERED TO BE RELEVANT

| Category* | Citation of document, with indication, where appropriate, of the relevant passages | Relevant to claim No. |
|---|---|---|
| X<br>A | BENITEZ, M. S., GARDENER, B. B. M., Linking sequence to function in soil bacteria: sequence-directed isolation of novel bacteria contributing to soilborne plant disease suppression, Applied and Environmental Microbiology, 2009, vol. 75, no. 4, pp. 915-924, abstract, fig. 1 | 1-2, 4-13<br>3 |
| P, X | MARIAN, M. et al., Novel strains of mitsuaria chitosanitabida and ralstonia pickettii as biocontrol agents against tomato bacterial wilt, 日本植物病理学会大会プログラム・講演要旨予稿集, 12 April 2017, pp. 95, 322, (Abstracts of PSJ Annual Meeting) | 1-13 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012211124 A

**Non-patent literature cited in the description**

- *Japanese Journal of Phytopathology,* 2016, vol. 82 (3), 246

- *The National Institute of Technology and Evaluation, NITE Patent Organism Depositary,* 17 March 2017